# EUROPEAN PATENT SPECIFICATION

(11) **EP 4 504 405 B1**
(45) Date of publication and mention of the grant of the patent: **04.03.2026**
(21) Application number: 23925619.1
(22) Date of filing: 08.03.2023
(51) Int. Cl.: B01J 38/00, C07C 69/54

(54) **ORGANOTIN CATALYSTS RECOVERY PROCESS**
VERFAHREN ZUR WIEDERGEWINNUNG VON ORGANOZINNKATALYSATOREN
PROCÉDÉ DE RÉCUPÉRATION DE CATALYSEURS A BASE D'ORGANOÉTAIN

(43) Date of publication of application: 12.02.2025
(73) Proprietor: SNF Group, 42160 Andrézieux-Bouthéon (FR)
(72) Inventor: FAVERO, Cedrick, 42160 Andrézieux, Bouthéon (FR); LEGRAS, Benoit, 42160 Andrézieux, Bouthéon (FR); KIEFFER, Johann, 42160 Andrézieux, Bouthéon (FR); BOISSE, Nicolas, Taizhou Jiangsu 225400 (CN); LING, Jing, Taizhou Jiangsu 225400 (CN)
(74) Representative: Ipsilon
(86) International application number: PCT/CN2023/080352
(87) International publication number: WO 2024/183039

(56) References cited:
- WO-A1-03/028888
- JP-A- 2000 159 726
- JP-A- 2003 211 200
- JP-A- 2011 126 836
- US-A- 4 382 864
- US-A- 6 087 528
- US-A- 6 111 054

## Description

### TECHNICAL FIELD OF THE INVENTION

The present invention relates to a new organotin catalysts recycling method carried out after an amino alkyl (meth)acrylates synthesis process.

### TECHNICAL BACKGROUND

Amino alkyl (meth)acrylates, such as 2-dimethylaminoethyl (meth)acrylate and its quaternized derivatives, are well-known monomers used in the preparation of polymers used in many industries, such as water treatment, papermaking, home and personal care, or oil & gas industry such as enhanced oil recovery, hydraulic fracturing, water shut-off and others.

These monomers are obtained by transesterification of alkyl(meth)acrylate with an amino alcohol, catalyzed by organometallic derivatives. Choosing the catalyst depends on various criteria, like the nature of the amino alkyl (meth)acrylate or of the alcohol used, or the nature of the synthesis process.

For 2-dimethylaminoethyl (meth)acrylate, it is well-known to use organotin derivatives as catalysts, in particular dialkyl tin oxide like dibutyl tin oxide (DBTO). The catalyst's role is to displace the equilibrium to produce more 2-dimethylaminoethyl (meth)acrylate and reduce the impurities formation. After the reaction, the catalyst can be recycled in a new synthesis process, but during these recycling cycles, the DBTO catalyst tends to be deactivated and it is necessary to replace it regularly, which is costly and generates toxic wastes difficult to manage and treat.

When DBTO starts to be deactivated, the reaction product contains more impurities. These impurities are Michael adducts of alcohols, such as methanol or dimethylaminoethanol which react with methyl(meth)acrylate, dimethylaminoethyl(meth)acrylate, or (meth)acrylic acid. These impurities, created as by-products, decrease the quality of the 2-dimethylaminoethyl (meth)acrylate produces. This decrease in monomer quality impacts the performance of the polymers prepared from such monomers.

Many attempts have been made to recycle the catalyst as efficiently as possible.

Document JP2008-231003 describes the synthesis of multi-functional acrylate and the recovery of the catalyst by addition of a strong acid.

Document WO03/028888 describes the recovering of a transesterification catalyst by extraction with water. US6087528A discloses recovering organotin reaction catalyst from a liquid reaction product by extraction.

Document WO2019/196048 describes a method for producing 2-dimethylaminoethyl (meth)acrylate using a mixture of fresh catalyst along with recycled catalyst.

As an alternative to organotin catalyst, tetraalcoxy titanate catalyst have been used for the synthesis of 2-dimethylaminoethyl (meth)acrylate as described in US 7,268,251 Such a titanium catalyst requires an activation step by firstly reacting tetraisopropyl titanate with dimethylaminoethanol for ligand exchange, thus generating side stream of isopropanol which has to be further disposed. This activation step is highly energy consuming and generates a waste side stream.

Despite all these, more or less successful, attempts, there is still a need for an improved process for catalyst recovery, to allow more recycling, and thus a reduction of the environmental impact of the amino alkyl (meth)acrylate synthesis process.

The recycling method according to the invention is in line with the principle of environmental consciousness and the impact of industry and man on the planet. The recycling method allows a better recovery of the catalyst and improved performances in the subsequent amino alkyl (meth)acrylates synthesis process leading to an overall reduction of the emission of greenhouse gases such as CO₂.

### SUMMARY OF THE INVENTION

The present invention relates to a new organotin catalysts recycling method after an amino alkyl (meth)acrylates synthesis process, for instance 2-dimethylaminoethyl (meth)acrylate.

More specifically, the organotin catalysts recycling method comprises the following successive (i.e. consecutive or succeeding) steps:
1) Preparation of a mixture comprising (i) a polar solvent selected from water, at least one C₁-C₁₂ alcohol, and a mixture of water and at least one C₁-C₁₂ alcohol (ii) an organotin catalyst residue solution S₁ resulting from the synthesis of an amino alkyl (meth)acrylate compound and (iii) at least one organic extraction solvent ES₁, liquid-liquid extraction LLE₁ of this mixture using the organic extraction solvent ES₁ to obtain an organic solution OS₁ and an aqueous solution AS₁;
2) Distillation D₁, at a temperature T₁ and at a pressure P₁, of the organic solution OS₁ to obtain an organic solution OS_{1'} and an organic distillate OD₁;
3) Distillation D₂, at a temperature T₂ and at a pressure P₂, of the organic solution OS₁, to obtain an organic solution OS_{1"} and an organic distillate OD₂, wherein the organic solution OS_{1"} contains 2 to 40 wt% of organotin catalyst residue;

the temperature T₂ being greater than the temperature T₁ and/or the pressure P₂ being lower than the pressure P₁;
wherein in step 1), the (ii) organotin catalyst residue solution S₁ and the (iii) at least organic extraction solvent ES₁ are already mixed when the (i) polar solvent is added or the (i) polar solvent, the (ii) organotin catalyst residue solution S₁ and the (iii) at least organic extraction solvent ES₁ are added simultaneously.

Disclosed herein is also an amino alkyl (meth)acrylate synthesis process using at least part of the recycled organotin catalyst obtained according to the recycling method of the invention.

Disclosed is further a polymer obtained from an amino alkyl (meth)acrylate synthesis process using at least part of the recycled organotin catalyst obtained according to the recycling method of the invention.

### DESCRIPTION OF THE INVENTION

As used herein, the expression "A and/or B" means "A, or B, or A and B".

As used herein, the expression heavie, ccorresponds to Michael adducts of alcohols, such as methanol or dimethylaminoethanol which react with methyl(meth)acrylate, dimethylaminoethyl(meth)acrylate, or (meth)acrylic acid.

### Liquid-liquid extraction LLE₁ of an organotin catalyst residue solution S₁

The recycling method can be carried out after any amino alkyl (meth)acrylate synthesis process as described in the prior art, using an alkyl (meth)acrylate and 2-dimethylaminoethanol as starting materials. Advantageously, the alkyl (meth)acrylate has an alkyl chain from 1 to 6 carbons (preferably a linear alkyl chain), more preferentially the alkyl(meth)acrylate is methyl(meth)acrylate. Preferably, the recycling method is carried out after a 2-dimethylaminoethyl (meth)acrylate synthesis process involving an organotin catalyst. The organotin catalyst residue solution S₁ therefore preferably results from the synthesis of 2-dimethylaminoethyl (meth)acrylate.

In an amino alkyl (meth)acrylate synthesis process, the reaction is carried out in presence of a reaction solvent which forms an azeotrope with the lower alcohols that are formed as by-products during the synthesis. This azeotrope is distilled during the synthesis process to shift the reaction to the formation of the amino alkyl (meth)acrylate.

The reaction solvent is advantageously selected from linear, branched or cyclic hydrocarbon, saturated or unsaturated, containing from 3 to 16 carbons. Preferably, the solvent is selected from n-hexane, the isomers of n-hexane, and mixtures thereof.

At the end of the amino alkyl (meth)acrylate synthesis process, as the azeotrope mixture has been distilled, a reaction mixture is obtained generally comprising the amino alkyl (meth)acrylate, residues of the catalyst (organotin catalyst) and traces of raw material such as alkyl (meth)acrylate, 2-dimethylaminoethanol, alcohol and reaction solvent. Generally, a distillation step is performed to separate the different compounds. After such distillation step, an organotin catalyst residue solution S₁ is obtained comprising the catalyst and high boiling compounds such as Michael adducts.

According to a preferred embodiment, the organotin catalyst residue solution S₁ results from the synthesis of an amino alkyl (meth)acrylate (preferably 2-dimethylaminoethyl (meth)acrylate) in the presence of dialkyl tin oxide, preferably dibutyl tin oxide.

According to the invention, in a first step (liquid-liquid extraction LLE₁ step), the (ii) organotin catalyst residue solution S₁ is mixed with at least an (iii) extraction solvent ES₁ and a (i) polar solvent selected from water or at least one C₁-C₁₂ alcohol or a mixture of water and at least one C₁-C₁₂ alcohol.

Generally, the organotin catalyst residue solution S₁ contains Michael adducts, high boiling compounds, an organotin catalyst and possibly traces of starting materials and amino alkyl (meth)acrylate.

The organotin catalyst residue solution S₁ comprises advantageously between 1 and 40 weight % of organotin catalyst, preferably between 3 and 30 weight %, more preferably between 5 and 20 weight %.

The organic extraction solvent ES₁ is advantageously selected from linear and saturated hydrocarbons containing from 3 to 16 carbons, linear and unsaturated hydrocarbons containing from 3 to 16 carbons, branched and saturated hydrocarbons containing from 3 to 16 carbons, branched and unsaturated hydrocarbons containing from 3 to 16 carbons, cyclic and saturated hydrocarbons containing from 3 to 16 carbons, cyclic and unsaturated hydrocarbons containing from 3 to 16 carbons and mixtures thereof. Preferably, the extraction organic ES₁ solvent is a saturated alkane having a carbon chain ranging from C₃ to C₁₂. It may be a mixture of C₃ to C₁₂ alkanes.

Examples of organic extraction solvents ES₁ used in the invention include n-propane, n-butane, n-pentane, n-hexane, n-heptane, n-decane, toluene, benzene, xylene, cyclohexane, isooctane, isomers of the C₃-C₆ and C₁₀ n-alkanes, and mixtures thereof.

Preferably, the organic extraction ES₁ solvent is chosen from n-propane, n-butane, n-pentane, n-hexane, n-heptane, n-decane, their isomers and mixtures thereof. More preferably, the organic extraction ES₁ solvent contains at least 50 weight % of n-hexane, even more preferably at least 60 weight%, even more preferably at least 70 weight%, even more preferably at least 80 weight%, and even more preferably at least 85 weight% of n-hexane.

As already mentioned, step 1) involves a polar solvent chosen from water, lower alcohols (C₁-C₁₂) and mixtures thereof. Lower alcohols (C₁-C₁₂) include methanol, ethanol, and mixtures thereof. Preferably, this polar solvent contains at least 70 weight % of water, more preferably at least 80 weight%, even more preferably at least 90 weight%.

In step 1), the weight ratio between the organic extraction solvent ES₁ and the polar solvent is advantageously comprised between 20:1 and 2:1, preferably between 15:1 and 2,5:1, more preferably between 12:1 and 3:1, even more preferably between 9:1 and 4:1.

The liquid-liquid extraction LLE₁ can be carried out by any means known by the skilled man in the art.

Advantageously, the liquid-liquid extraction LLE₁ comprises at least: one mixing step and one separation step.

The liquid-liquid extraction LLE₁ is advantageously carried out at a temperature comprised between 1 and 90°C, preferably between 10 and 80°C, more preferably between 20 and 70°C.

In a particular embodiment, the LLE₁ is performed using a continuous extraction column.

The mixing step (polar solvent + ES₁ + S₁) can be carried out using any mixing equipment, for example, with a stirring blade, a centrifugal pump, a static mixer, a rotor/stator combination. Preferably, the mixing step is carried out using a stirring blade. It involves mixing (i) a polar solvent selected from water, at least one C₁-C₁₂ alcohol, and a mixture of water and at least one C₁-C₁₂ alcohol (ii) an organotin catalyst residue solution S₁ resulting from the synthesis of an amino alkyl (meth)acrylate compound and (iii) at least one organic extraction solvent ES₁.

The mixing time (polar solvent + ES₁ + S₁) advantageously lasts between 5 seconds and 60 minutes, preferably between 1 minutes and 30 minutes, more preferably between 90 seconds and 10 minutes.

The rotation speed for the mixing (polar solvent + ES₁ + S₁) is advantageously comprised between 10 and 10 000 rpm, preferably between 100 and 5 000 rpm, more preferably between 200 and 1 000 rpm (rpm = rotation per minute).

The mixing time and the rotation speed (polar solvent + ES₁ + S₁) depend (1) on the agitator design and mixing vessel volume, (2) on the quantity of organotin catalyst in the organotin catalyst residue solution S₁, (3) on the ratio between the solvents (polar + ES₁) and the organotin catalyst residue solution S₁. The skilled man in the art is able to determine these parameters without difficulty, as they correspond to conventional settings.

The separation step allows the formation of two separate phases, an aqueous phase and an organic phase. The separation step can be achieved by any one of the following methods: centrifuge, coalescer, cyclone, column, gravity settling, and combinations thereof. Preferably, the separation step is achieved by gravity settling.

The separation step advantageously lasts between 10 seconds and 60 minutes, preferably between 30 seconds and 30 minutes, more preferably between 1 min minutes and 10 minutes.

At the end of the liquid-liquid extraction LLE₁, an organic solution OS₁ and an aqueous suspension AS₁ are obtained.

Generally, the organic solution OS₁ contains Michael adducts, the organotin catalyst residue, the organic extraction solvent ES₁ and possibly traces of amino alkyl (meth)acrylate,

The organic solution OS₁ advantageously comprises between 1 and 40 weight % of organotin catalyst residue, preferably between 2 and 30 weight %, more preferably between 3 and 20 weight %.

Generally, the aqueous suspension AS₁ contains some of the organotin catalysts and the starting materials, amino alkyl (meth)acrylate.

The aqueous suspension AS₁ advantageously comprises between 0,1 and 10 weight % of organotin catalyst residue, preferably between 0,15 and 5 weight %, more preferably between 0,5% and 2 weight %.

### Liquid-liquid extraction LLE₂ of the aqueous suspension AS₁

In a particular embodiment, the aqueous suspension AS₁ is treated by liquid-liquid extraction LLE₂, with an extraction solvent ES₂.

This liquid-liquid extraction LLE₂ can be carried out with the same equipment as describe for the liquid-liquid extraction LLE₁.

The organic extraction solvent ES₂ is advantageously an organic solvent.

The organic extraction solvent ES₂ is advantageously selected from linear and saturated hydrocarbons containing from 3 to 16 carbons, linear and unsaturated hydrocarbons containing from 3 to 16 carbons, branched and saturated hydrocarbons containing from 3 to 16 carbons, branched and unsaturated hydrocarbons containing from 3 to 16 carbons, cyclic and saturated hydrocarbons containing from 3 to 16 carbons, cyclic and unsaturated hydrocarbons containing from 3 to 16 carbons and mixtures thereof. Preferably, the organic extraction solvent ES₂ is a saturated alkane having a carbon chain ranging from C₃ to C₁₂. It may be a mixture of C₃ to C₁₂ alkanes.

Example of organic extraction solvent ESₙ (n is an integer greater than or equal to 1) are n-propane, n-butane, n-pentane, n-hexane, hexane, n-heptane, n-decane, toluene, benzene, xylene, cyclohexane, isooctane, their isomers and mixtures thereof, preferably the organic extraction solvent ESₙ is n-hexane and its isomers.

The ratio between the extraction solvent ES₂ and the aqueous suspension AS₁ is advantageously comprised between 20:1 and 2:1, preferably between 15:1 and 2,5:1, more preferably between 12:1 and 3:1, even more preferably between 9:1 and 4:1.

There is no particular addition order for the extraction solvent ES₂ and aqueous solution AS₁. Addition of the extraction solvent can be decomposed in several steps. The extraction solvent ES₂ can be added first, or the aqueous suspension AS₁ can be added first, they can be added alternately (i.e. a first fraction of the extraction solvent ES₂ followed by a first fraction of the aqueous suspension AS₁, then a second fraction of the extraction solvent ES₂ followed by a second fraction of the aqueous suspension AS₁, or vice versa) or they can be added simultaneously. Preferably, the extraction solvent ES₂ is added first.

The liquid-liquid extraction LLE₂ can be carried out by any means known by the skilled man in the art. Advantageously, the liquid-liquid extraction LLE₂ comprises at least one mixing step and one separation step.

The liquid-liquid extraction LLE₂ is advantageously carried out at a temperature comprised between 1 and 90°C, preferably between 10 and 80°C, more preferably between 20 and 70°C.

The mixing time advantageously last between 5 seconds and 30 minutes, preferably between 1 minutes and 10 minutes, more preferably between 90 seconds and 5 minutes.

The rotation speed for the mixing is advantageously comprised between 50 and 10 000 rpm, preferably between 100 and 5 000 rpm, more preferably between 200 and 1 000 rpm.

The mixing time and rotation speed depend (1) on the agitator design, the mixing vessel volume, (2) on the quantity of organotin catalyst residue in the aqueous suspension AS₁ and (3) on the ratio between the extraction solvent ES₂ and the aqueous solution AS₁. The skilled man in the art is able to determine these parameters without difficulty, as they are conventional settings.

The second separation step allows the formation of two separate phases, an aqueous and an organic phase. The separation step is advantageously a gravity settling.

The separation step advantageously lasts between 10 seconds and 60 minutes, preferably between 30 seconds and 10 minutes, more preferably between 1 minutes and 5 minutes.

At the end of the liquid-liquid extraction LLE₂, an organic solution OS₂ and an aqueous suspension AS₂ are obtained.

In a preferred embodiment, the organic solution OS₂ is recycled, partially or totally, so as to form or complete the organic extraction solvent ES₁ in the first liquid-liquid extraction LLE₁.

In a particular embodiment, the aqueous suspension AS₂ is discarded.

In a preferred embodiment, the aqueous suspension AS₂ is distilled, and the resulting aqueous distilled phase is recycled in the first liquid-liquid extraction LLE₁ or in the second liquid-liquid extraction LLE₂, partially or totally.

In a particular embodiment, at least one carboxylic acid is added to the aqueous suspension AS₁ before performing the liquid-liquid extraction LLE₂. The carboxylic acid is advantageously chosen from acrylic acid, methacrylic acid, acetic acid, formic acid, itaconic acid, maleic acid, citric acid, fumaric acid, tartaric acid and mixtures thereof. Preferably, the carboxylic acid is acrylic acid.

The weight ratio between the carboxylic acid and the organotin catalyst residue in the aqueous suspension AS₁ is advantageously comprised between 0.005 and 0.2, preferably between 0.01 and 0.15, more preferably between 0.02 and 0.1.

The liquid-liquid extraction LLE₂ is advantageously carried out at a temperature comprised between 1 and 200°C, preferably between 5 and 120°C, more preferably between 5 and 80, more preferably between 10 and 70°C.

The liquid-liquid extraction step is advantageously carried out at an absolute pressure comprised between 0.1 and 20 bars, preferably between 0.5 and 5 bars, more preferably between 0.8 and 2 bars, more preferably 0.9 and 1.1 bar, more preferably at atmospheric pressure.

Optionally, after LLE₁, a distillation step of AS₁ is performed to obtain an aqueous phase with a water content superior or equal to 90% by weight, said aqueous phase is advantageous recycled for a liquid-liquid extraction.

### Distillation D₁

In a second step, a distillation D₁ is carried out on the organic solution OS₁ to afford an organic solution OS_{1'}.

The distillation D₁ can be carried out in any distillation equipment known by the skilled man in the art, for example: a flash evaporator, a thin film evaporator, a wiped film evaporator, a shortpath distillation equipment, a falling film, a stirred reactor, or a kettle. Preferably, the distillation D₁ is carried out in a flash evaporator.

The distillation D₁ is advantageously carried out at a temperature T₁ comprised between 5 and 180°C, preferably between 10 and 150°C, more preferably between 15 and 80°C.

The distillation D₁ is advantageously carried out at an absolute pressure P₁ comprised between 0.01 and 1 bar, preferably between 0.05 and 0.8 bar, more preferably between 0.3 and 0.6 bar.

At the end of the distillation D₁, an organic solution OS_{1'} is obtained.

The organic solution OS_{1'} advantageously comprise between 1 and 40 weight % of organotin catalysts residue, preferably between 2 and 35 weight %, more preferably between 3 and 30 weight %.

Distillation D₁ results in a distillate (fraction DF₁) and in an organic solution OS_{1'}.

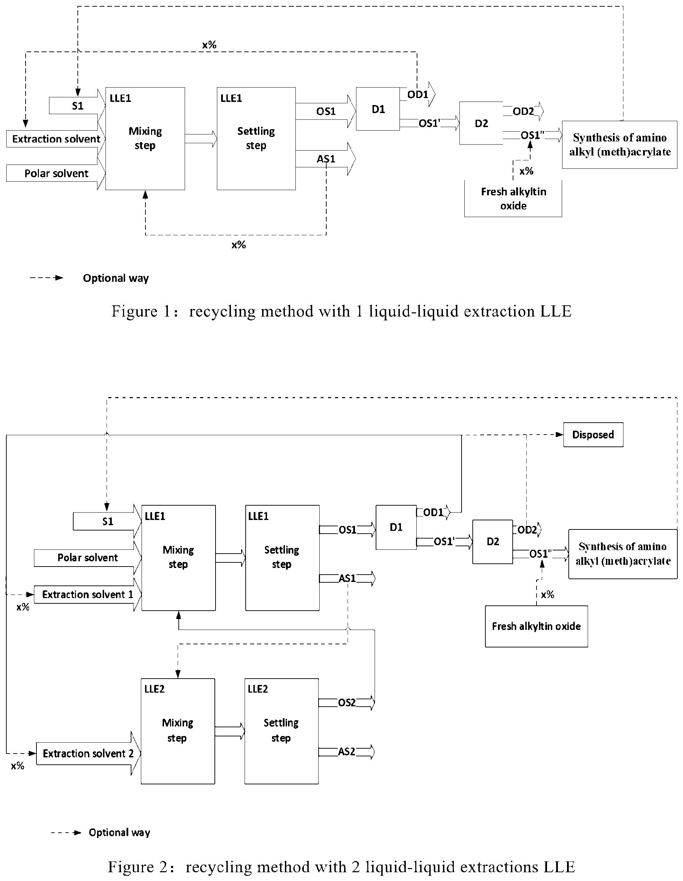

In a particular embodiment, the distilled fraction DF₁ can be recycled, partially or totally, with or without pre-treatment step for the first and/or second liquid-liquid extraction LLE₂ (as illustrated in Fig. 2).

In a particular embodiment, at least one carboxylic acid is added to the organic solution OS₁ before performing the distillation D₁. The carboxylic acid is advantageously chosen from acrylic acid, methacrylic acid, acetic acid, formic acid, itaconic acid, maleic acid, citric acid, fumaric acid, tartaric acid, and mixtures thereof. Preferably, the carboxylic acid is acrylic acid.

The weight ratio between the carboxylic acid and the organotin catalyst residue in the organic solution OS₁ is advantageously comprised between 0.005 and 0.2, preferably between 0.01 and 0.15, more preferably between 0.02 and 0.1.

### Distillation D₂

In a third step, a distillation D₂ is carried out on the organic solution OS_{1'} to afford an organic solution OS_{1"},
The distillation D₂ can be carried out in any distillation device known by the skilled man in the art, for example: a flash evaporator, a thin film evaporator, a wiped film evaporator, a shortpath distillation equipment, a falling film, a stirred reactor, or a kettle. Preferably, the distillation D₂ is carried out in a flash evaporator.

The distillation D₂ is advantageously carried out at a temperature T₂ comprised between 20 and 220°C, preferably between 30 and 200°C, more preferably between 50 and 190°C.

The distillation D₂ is advantageously carried out at an absolute pressure P₂ comprised between 0.001 and 1 bar, preferably between 0.01 and 0.5 bar, more preferably between 0.02 and 0.2 bar.

The temperature T₂ of distillation D₂ is greater than the temperature T₁ of distillation D₁ and/or the pressure P₂ of distillation D₂ is lower than the pressure P₁ of distillation D₁.

At the end of the distillation D₂, an organic solution OS_{1"} and a distillate fraction DF₂ are obtained.

The organic solution OS_{1"} advantageously comprises between 2 and 40 weight% of organotin catalyst residue, preferably between 3 and 40 weight %, more preferably between 5 and 40 weight %.

The distilled fraction DF₂ is generally composed of Michael adducts and the organic extraction solvent ESₙ (n is an integer greater or equal to 1) and the amino alkyl (meth)acrylate. The distilled fraction DF₂ can be disposed or further treated according to the method of document WO 2016/124837 describing a thermal treatment of high boiling compounds to reclaim and recycle 2-dimethylaminoethanol and/or alkyl (meth)acrylate and/or 2-dimethylaminoethyl (meth)acrylate.

In a particular embodiment, at least one carboxylic acid is added to the organic solution OS₁. before performing the distillation D₂. The carboxylic acid is advantageously chosen from acrylic acid, methacrylic acid, acetic acid, formic acid, itaconic acid, maleic acid, citric acid, fumaric acid, tartaric acid, and mixtures thereof. Preferably, the carboxylic acid is acrylic acid.

The weight ratio between the carboxylic acid and the organotin catalyst residue in the organic solution OS_{1'} is advantageously comprised between 0.005 and 0.2, preferably between 0.01 and 0.15, more preferably between 0.02 and 0.1.

At least part of the resulting organic solution OS_{1"} can be recycled as catalyst in an amino alkyl (meth)acrylates synthesis process. Advantageously, 100 weight% of the catalyst come from the recycled organic solution OS_{1"} based on the total amount of catalyst of the new amino alkyl (meth)acrylates synthesis process, preferably not more than 90 weight%, more preferably not more than 80 weight %, more preferably not more than 70 weight%, more preferably not more than 60 weight%, more preferably not more than 50 weight%. The remaining catalyst is advantageously fresh organotin catalyst.

By "fresh", we mean not recycled organotin catalyst.

### Optional step 4)

In a particular embodiment according to the invention, the organotin catalyst recycling method comprises an optional step 4).

The optional step 4) comprise at least an additional liquid-liquid extraction LLEₙ of aqueous suspension ASₙ₋₁, with an extraction solvent ESₙ to obtain an organic solution OSₙ and an aqueous suspension ASₙ with n is a non-zero integer > 1.

More specifically, the recycling method can include one or more of the following additional liquid-liquid extraction LLEₙ₊₁, wherein n is an integer greater than or equal to 1:
- Preparation of a mixture comprising (i) an aqueous suspension ASₙ (ii) at least one extraction solvent ESₙ,
liquid-liquid extraction LLEₙ₊₁ of this mixture to obtain an organic solution OS_{n+ 1} and aqueous suspension ASₙ₊₁.

In this particular embodiment, at least part of the organic solution OSₙ₊₁ is distilled according to steps 2) and 3) of claim 1, to respectfully afford an organic solution OS'ₙ₊₁ and then an organic solution OS''ₙ₊₁.

At least part of the resulting organic solution OS''_{n+1'} can be used as catalyst for an amino alkyl (meth)acrylates synthesis process. Advantageously, 100 weight% of the catalyst of this synthesis process come from the recycled organic solution OS"ₙ₊₁ based on the total amount of catalyst, preferably not more than 90 weight%, more preferably not more than 80 weight %, more preferably not more than 70 weight%, more preferably not more than 60 weight%, more preferably not more than 50 weight%. The remaining catalyst is advantageously fresh organotin catalyst.

In this particular embodiment, the aqueous suspension ASₙ₊₁ is use partially or totally, with or without pre-treatment step for a liquid-liquid extraction LLEₙ₊₂ and the organic solution OSₙ₊₁ is recycled partially or totally, with or without pre-treatment step in a liquid-liquid extraction LLEₙ.

In this particular embodiment, the extraction solvent ESₙ is advantageously composed of at least one organic solvent, advantageously selected from linear, branched or cyclic hydrocarbon, saturated or unsaturated, containing from 3 to 16 carbons, preferably from saturated alkane having a carbon chain ranging from C₃ to C₁₂, and mixture thereof, wherein the total amount of polar solvent and organic solvent corresponds to 100 weight% of the extraction solvent ESₙ.

Examples of organic extraction solvent used for the extraction solvent ESₙ include n-propane, n-butane, n-pentane, n-hexane, hexane, n-heptane, n-decane, toluene, benzene, xylene, cyclohexane, isooctane, their isomers and mixtures thereof.

In a particular embodiment, the aqueous suspension ASₙ₊₁, is advantageously distilled (D₃) to obtain an aqueous suspension AS_{n+1'} and an organic solution OS_{n+1'}.

The distillation D₃ can be carried out in any distillation equipment known by the skilled man in the art, for example: a flash evaporator, a thin film evaporator, a wiped film evaporator, a shortpath distillation equipment, a falling film, a stirred reactor, or a kettle. Preferably, the distillation D₃ is carried out in a flash evaporator.

The distillation D₃ is advantageously carried out at a temperature T₃ comprised between 10 and 100 °C, preferably between 30 and 100°C, more preferably between 50 and 100°C.

The distillation D₃ is advantageously carried out at an absolute pressure P₃ comprised between 0.001 and 1 bar, preferably between 0.05 and 1 bar, more preferably between 0.1 and 1 bar.

In a particular embodiment the organic solution OSₙ₊₁ is advantageously recycled, partially or totally, with or without pre-treatment step for the LLEₙ.

### Particular embodiments of the invention

In a particular embodiment, the distillation D₁ and the distillation D₂ are carried out in the same distillation device.

In a particular embodiment, at least one carboxylic acid and/or one alcohol is added to the organic solution OS_{1"} before recycling it for the synthesis of amino alkyl (meth)acrylate synthesis.

The carboxylic acid is advantageously chosen from acrylic acid, methacrylic acid, acetic acid, formic acid, itaconic acid, maleic acid, citric acid, fumaric acid, tartaric acid, and mixtures thereof. Preferably, the carboxylic acid is acrylic acid.

The alcohol is advantageously chosen from lower alcohol (C₁-C₁₂), for example methanol, ethanol, propanol, butanol, and mixtures thereof. Preferably, the alcohol is methanol.

In the organic solution OS_{1"}, the molar ratio between (1) the carboxylic acid and/or the alcohol and (2) the organotin catalyst residue is advantageously comprised between 1:50 and 1:2, preferably between 1:30 and 1:5, more preferably between 1:20 and 1:10.

The recycling method can be carried out in batch or as a semi continuous or continuous process. Preferably, the recycling method is carried out as a continuous process.

In a particular embodiment, the recycling method of the invention is carried out as a continuous process. By "continuous process" according to the invention, it is to be understood a process in which at least one stream enters continuously (ESₙ), advantageously the organotin catalyst residue solution S₁, and from which at least two streams leave continuously, advantageously one LS₁ corresponding to the aqueous phase ASₙ and one LS₂ corresponding to the organic phase OSₙ (n is an integer greater than or equal to 1). The continuous process according to the invention can operate from several days to several months without interruption.

In this particular embodiment, the resident time within the distillation device (D₁+D₂) is comprised between 30 seconds and 60 minutes, preferably between 1 minutes and 30 minutes, more preferably between 5 minutes and 20 minutes.

In this particular embodiment, the weight ratio between LS₁ and LS₂ is advantageously comprised between 0.01 and 1, preferably between 0.1 and 1, more preferably between 0.15 and 1 (n is an integer greater than or equal to 1).

Disclosed is further an amino alkyl (meth)acrylate synthesis process using at least part of the recycled organotin catalyst obtained according to the recycling method of the invention.

Advantageously, at least not more than 70 weight% of the catalyst used in this synthesis come from the organotin catalysts recycling method of the invention, based on the total amount of catalyst, preferably not more than 60 weight%, more preferably not more than 50 weight%. The remaining catalyst is advantageously fresh organotin catalyst.

Polymer is obtained from an amino alkyl (meth)acrylate synthesis process using at least part of the recycled organotin catalyst obtained according to the recycling method of the invention.

The invention and the advantages thereof will be better illustrated by, but not limited to, the following examples given to illustrate the invention.

### FIGURES DESCRIPTION

[Fig. 1] Figure 1 represents a continuous mode with 1 liquid-liquid extraction LLE.
[Fig. 2] Figure 2 represents a continuous mode with 2 liquid-liquid extractions LLE.
[Fig. 3] Figure 3 represents a continuous mode with n liquid-liquid extractions LLE.

### List of abbreviations:

ADAME: 2-dimethylaminoethyl acrylate
MA: Methylacrylate
DMOH: Dimethylaminoethanol
Hx: Hexane
DBTO: Dibutyl tin oxide
Ptz: Phenothiazine
GC-FID: Gas chromatography flame ionization detector
Hv: Heavie
MeOH: Methanol
ICP-OES: Inductively coupled plasma - optical emission spectroscopy
AZDN: Azobisisobutyronitrile

### EXAMPLES

### Example 1 - ADAME synthesis

In a 250 mL three-neck round flask, 89 g of MA, 46 g of DMOH, 13g of Hx, 1.84g of DBTO and 0.11g of Ptz are added. A magnetic stirrer is started at 500 RPM.

On one neck, a 50 cm heighted column, 20 mm inner diameter packed with Dixon rings 3 mm is mounted with on the top of the column a cooling condenser with glycol water at -10°C.

The top column temperature is monitored and adjusted by changing the reflux ratio, a metering valve is used to control the distillate liquid collection.

A heated plate is used to heat the round flask, the external temperature is set to 110°C. Distillate is continuously collected at the top of the column by maintaining the top temperature from 48 to 53°C using the reflux ratio control valve.

Every 10 mL of distillate collected the reacting medium is reloaded with 7 mL of Hx. The reaction is pursued until the top of the temperature column cannot be maintained below 53.5°C by increase the reflux ratio to full.

The reaction mixture is analysed with a GC-FID.

A GC Agilent 7820A is used with a DB-WAX UI column of 30m and 0.50µm as inner diameter.

The temperature is set at 80°C for 5 min, then ramp up at 4°C per min to 122°C and set at 122°C for 2 min, and then ramp up again at 35°C per min to 240°C and set à 240°C for 9 min. The injector is set at 250 °C, helium is used as phase carrier. 1 µL is injected with 1: 100 split ratio.

A flame ionisation detector is used for the detection.

5 sample standards with different compositions are injected with ADAME, DMOH, MA, MeOH, Hx to establish the calibration curves. The response factor got for ADAME is used for the heavies's quantification after 16 min retention time.

It measured 64.6%wt ADAME concentration, 0.9wt% for DMOH, and high molecular weight heavies are measured to be at 0.35%.

The ADAME conversion is calculated as ((% ADAME/(% ADAME+% DMOH+% Heavies))*100) = 98.2%.

The reaction mixture is then distilled under vacuum using the same setup to recover the ADAME monomer. The different fractions are subsequently collected with the final ADAME fraction when the top column temperature reach 78°C at 40 mbars until 10 mbars absolute.

The composition of the ADAME fraction is 99.5%wt ADAME, 1500 ppm DMOH, 100 ppm MeOH, MA 1500 ppm and Hv 505 ppm, measured by GC-FID.

The concentrated solution in the round flask corresponds to the catalyst residue solution S₁.

The tin concentration is measured by ICP-OES with a ICPE-5800 Agilent. 0.1g of S₁ is weighted in 8 ml concentrate chlorohydric acid and 2 ml nitric acid. The sample is placed in a closed crucible. The crucible is heated in a microwave at 600 W for 90 min to reach 150°C. The solution obtained is diluted 100 times and nebulised at 0.7L/min. Emission intensity is measured at 189,925 nm wavelength for quantification, based on tin standard solution.

The tin concentration is corrected with the molecular weight assuming a pure DBTO structure. 2.87%wt of Sn is measured corresponding to 6%wt of DBTO.

### Example 2 (Invention)

In a 1L reactor equipped with a mechanical stirrer defined by a 4-bladed 90° impeller. 500 g of Hx and 250 g of the catalyst residue solution S₁ prepared in example 1 are added.

The stirrer is set to 500 RPM, and after 30 seconds 50 g of water is added.

After 1 minute the stirring is stopped and the solution is left to settle for 1 minute. The top and bottom phases are separately collected, they correspond respectively to OS₁ and AS₁.

In a 1L batch distillation apparatus the OS₁ is distilled for 30 min at 60°C; 300 mbars to collect a Hx phase in distillate DF₁, the bottom phase corresponds to OS_{1'}.

Then the distillation is pursued at 120°C; 5 mbars until no distillate can be collected. 35g are obtained in the bottom concentrate corresponding to OS_{1"}.

The tin composition in OS_{1"} is measured per ICP-OES to be at 6%wt, corresponding to 12.6%wt of DBTO.

### Example 3 (Counter example)

In a 1L reactor equipped with a mechanical stirrer defined by a 4-bladed 90° impeller. 500 g of Hx and 250 g of the catalyst residue solution S₁ prepared in example 1 are added.

The stirrer is set to 500 RPM, after 30 seconds 50 g of water are added.

After 1 minute the stirring is stopped and the solution is left to settle for 1 minute. The top and bottom phases are separately collected, they correspond respectively to OS₁ and AS₁.

In a 1L batch distillation apparatus the OS₁ is distilled for 30 min at 60°C; 300 mbars to collect a Hx phase in distillate DF₁.

55g is obtained in the bottom concentrate corresponding to OS_{1'}.

The tin composition in OS_{1'} is measured per ICP-OES to be at 3.80%wt, corresponding to 8.0%wt of DBTO.

### Example 3-2 (Counter example)

In a 1L reactor equipped with a mechanical stirrer defined by a 4-bladed 90° impeller. 50 g of water and 250 g of the catalyst residue solution S₁ prepared in example 1 are added.

The stirrer is set to 500 RPM, after 30 seconds 500 g of Hx are added.

After 1 minute the stirring is stopped and the solution is left to settle for 1 minute. The top and bottom phases are separately collected, they correspond respectively to OS₁ and AS₁. In a 1L batch distillation apparatus the OS₁ is distilled for 30 min at 60°C; 300 mbars to collect a Hx phase in distillate DF₁. Then the distillation is pursued at 120°C; 5 mbars until no distillate can be collected. 45g are obtained in the bottom concentrate corresponding to OS_{1"}.

The tin composition in OS_{1"} is measured per ICP-OES to be at 0.5%wt, equivalent to 1.05%wt of DBTO.

### Example 3-3 (Counter example)

In a 1L reactor equipped with a mechanical stirrer defined by a 4-bladed 90° impeller. 500 g of Hx and 250 g of the catalyst residue solution S₁ prepared in example 1 are added.

The stirrer is set to 500 RPM, and after 30 seconds 300 g of water is added.

After 1 minute the stirring is stopped and the solution is left to settle for 1 minute.

A stable emulsion is obtained. The bottom and top phase cannot be separate.

### Example 4 (Invention)

In a 1L reactor equipped with a mechanical stirrer defined by a 4-bladed 90° impeller. 500 g of Hx and 250 g of the catalyst residue solution S₁ prepared in example 1 are added.

The stirrer is set to 500 RPM, after 30 seconds 50 g of water are added.

After 1 minute the stirring is stopped and the solution is left to settle for 1 minute. The top phase corresponding to OS₁ is collected.

The bottom phase corresponding to AS₁ is let in the reactor and 500 g of Hx is added followed per 1 minute stirring at 500 RPM and 1 minute settling.

The top and bottom phases are separately collected, they correspond respectively to OS₂ and AS₂.

In a 1L batch distillation apparatus the OS₂ is distilled for 30 min at 60°C; 300 mbars to collect a Hx phase in distillate DF1, the bottom phase corresponds to OS_{2'}.

Then the distillation is pursued at 120 °C; 5 mbars until no distillate can be collected. 40g are obtained in the bottom concentrate OS_{2"}.

The tin composition in OS_{2"} is measured per ICP-OES to be at 7.3%wt, corresponding to 15.4 %wt of recycled DBTO.

### Example 5 (Invention)

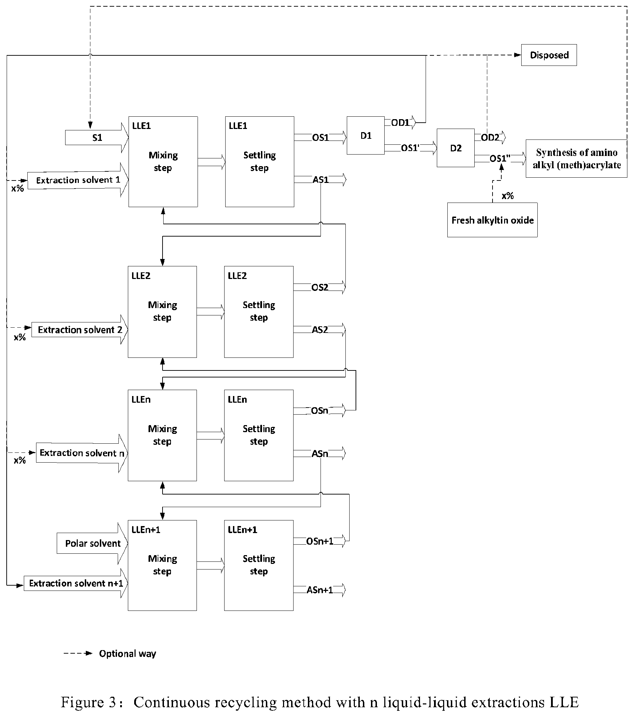

This example corresponds to the particular mode according to figure 3.

In a 7 L Kühni agitated columns composed with a 10 level of 4-bladed 90° impellers, 6L Hx is loaded.

The stirrer is set to 100 RPM. 250 g/h of the catalyst residue solution S₁ prepared in example 1 are continuously added on the first tray of the column.

A homogeneous mixture of Hx/water (50:50) is continuously added at the second tray next to the top of the column at a rate of 100 g/h.

At the last tray of the column the Hx is continuously added at 200 g/h.

At the top and bottom of the column, are respectively collected the organic phase OS₁ and the aqueous phase AS₁.

In a rotavapor apparatus the OS₁ is distilled for 30 min at 60°C; 300 mbars to collect a Hx phase in distillate DF₁, and the organic phase OS_{1'} at the bottom.

Then the distillation is pursued one OS_{1'} at 120 °C; 5 mbars until no distillate can be collected.

45g/h are obtained in the bottom concentrate OS_{1"},

The tin composition in OS_{1"} is measured using ICP-OES to be at 8.1%wt, corresponding to 17 %wt of recycled DBTO.

### Example 6 - ADAME with the recycled DBTO of examples 2-5

The recycled DBTO produced in example 2 to 5 are used in a new ADAME synthesis process as described in example 1, except the catalyst quantity is adjusted to start with the same DBTO concentration.

The final composition of the ADAME fraction is summarised in the table 1.

The composition of the ADAME fraction is 99.5%wt ADAME, 1500 ppm DMOH, 100 ppm MeOH, MA 1500 ppm and Hv 505 ppm, measured by GC-FID.

**Table 1**

| | **Ex1 (Reference)** | **Ex2 (Invention)** | **Ex3 (Counter example)** | **Ex4 (Invention)** | **Ex5 (Invention)** |
|---|---|---|---|---|---|
| **OS_{1'} or OS_{1"}** | | 14.6 g | 23.0 g | 11.9 g | 10.8 g |
| **ADAME conversion based on DMOH** | 98.2% | 97.5% | 97.0% | 98.1% | 97.5% |
| **Hv in reactor prior ADAME distillation** | 0.35% | 0.5% | 1.0% | 0.55% | 0.45% |
| **ADAME purity after distillation** | 99.5% | 99.5% | 99.3% | 99.6% | 99.5% |
| **Hv in ADAME fraction** | 505 ppm | 525 ppm | 630 ppm | 536 ppm | 510 ppm |

### Example 7 - ADAME polymer synthesis and application tests

In the following example the ADAME material synthetised in the example 6 are quaternized with methyl chloride.

In a 2L autoclave reactor equipped with 4-blades impeller and a double-jacket, the reactor is degassed with a nitrogen flowrate for 30 min then closed.

Using a gear pump ADAME is added at rate of 236 g/h for 3h. When 200 ml is reached, the reactor stirring is started and methyl chloride is added at rate of 128 g/h for 2 h. The temperature is regulated to reach a maximum of 45°C using the double-jacket. After 1h the water addition is started at a rate of 216 g/h for 1h.

The mixture is left stirred for 1h at 40°C, then cooled down and degassed to reach the atmosphere pressure.

The chloromethylated ADAME(ADC80) is then diluted with 10 g of water.

1190g of finished product is obtained at an active concentration of 80 weight%. The finished product is then polymerised as described in the following process:
In a 2L beaker, 137 g of acrylamide solution (50%wt in water), 437 g of water and 926 g of ADC80 are added.

The mixture is cooled to 0°C under stirring and the pH is adjusted with phosphoric acid to a value of 3.6.

The solution is transferred to a Dewar pot and degassed with nitrogen for 15 min. 1 mg in 1 mL of water of AZDN is then added.

The nitrogen degassing is maintained for 15 min and the temperature is monitored.

At the end of the degassing period, 2 mg sodium hypophosphite in 1mL water is added to the Dewar pot, followed with 6 mg of sodium persulfate in 1 mL water and 4 mg of Mohr salt dissolved in 1 mL water. The polymerisation reaction starts.

The reaction is then left thermally insulated until the temperature reaches a plateau between 80 and 85°C.

The gel obtained is then left for 30 min at the plateau temperature and then is grinded in small pieces.

The grinded material is dried in an oven for 2h at 70°C.

The polymer particles are then crushed to powder with a particle size ranging from 1.5 to 2.5 mm.

The UL viscosity is measured using a Brookfield viscometer equipped with a UL adapter, at 60 revolutions/minute (0.1 percent by weight of polymer in a 1M saline sodium chloride solution) between 23 and 25°C.

The insolubility is measured by transferring 1g of the polymer solution in 200 ml of water at 20°C, stirring for 2h, then the dissolved solution is filtered with a 4 cm diameter filter with a porosity of 200µm and the solution is drained on.

The insolubility number corresponds to the number of aggregates on the filter, after the whole solution is passed through the filter, visually counted. The results are summarized in table 2.

**Table 2**

| **Polymer using the monomer produced in** | **Ex1 (Ref)** | **Ex2 (Invention )** | **Ex3 (Counter example)** | **Ex4 (Invention )** | **Ex5 (Invention )** |
|---|---|---|---|---|---|
| **UL viscosity (cps)** | 5.20 | 5.30 | 5.00 | 5.30 | 5.25 |
| **Insoluble number** | 0 | 0 | 5 | 0 | 0 |

### Example 8 - ADAME synthesis from example 1

The catalyst residue solution S₁ produced in example 1 is directly reused without any treatment in an ADAME synthesis process as described in example 1 excepted the raw materials methyl acrylate, dimethyl amino ethanol are adjusted referring to the residual monomer in the catalyst residue solution S₁. The operation is repeated 10 times. The results are summarized in table 3.

**Table 3**

| **Example** | **8** | **8.1** | **8.2** | **8.3** | **8.4** | **8.5** | **8.6** | **8.7** | **8.8** | **8.9** |
|---|---|---|---|---|---|---|---|---|---|---|
| **Number of synthesis** | 1 (example 1) | 2 | 3 | 4 | 5 | 6 | 7 | 8 | 9 | 10 |
| **ADAME conversion based on DMOH %** | 98.2 | 95.0 | 92.2 | 89.5 | 88.7 | 89.2 | 85.2 | 85.2 | 80.7 | 75.2 |
| **Hv in reactor prior ADAME distillation %** | 0.35 | 1.2 | 1.9 | 2.3 | 2.9 | 4 | 4.9 | 5.5 | 6.4 | 7.2 |
| **ADAME purity after distillation %** | 99.5 | 99.3 | 99.6 | 99.0 | 98.5 | 98.3 | 98 | 98.1 | 98.0 | 97.9 |
| **Hv in ADAME fraction ppm** | 505 | 630 | 720 | 900 | 2100 | 2500 | 3200 | 4000 | 7000 | 10000 |

### Example 9 - ADAME synthesis with the recycling method of example 4 between each cycle

The organic solution OS_{1"} produced in example 4 is used in a new process as described in example 1 excepted the raw materials methyl acrylate, dimethylaminoethanol are adjusted referring to the residual monomer in the organic solution OS_{1"}. The operation is repeated 10 times. The results are summarized in table 4.

| **Example** | **9** | **9.1** | **9.2** | **9.3** | **9.4** | **9.5** | **9.6** | **9.7** | **9.8** | **9.9** |
|---|---|---|---|---|---|---|---|---|---|---|
| **Number of synthesis** | 1 (example 1) | 2 (example 4) | 3 | 4 | 5 | 6 | 7 | 8 | 9 | 10 |
| **ADAME conversion based on DMOH %** | 98.2 | 98.1 | 96.0 | 95.0 | 95.0 | 96.0 | 95.0 | 96.0 | 96.5 | 95.0 |
| **Hv in reactor prior ADAME distillation %** | 0.35 | 0.55 | 0.75 | 1 | 1.35 | 1.55 | 1.75 | 2.00 | 2.4 | 2.7 |
| **ADAME purity after distillation %** | 99.5 | 99.6 | 99.6 | 99.4 | 99.5 | 99.4 | 99.4 | 99.4 | 99.3 | 99.3 |
| **Hv in ADAME fraction ppm** Table 4 | 505 | 536 | 750 | 820 | 870 | 920 | 1050 | 1200 | 1450 | 2010 |

### Example 10 - ADAME synthesis with the recycling method of example 4 between each cycle mixed with 50% fresh catalyst

The organic solution OS_{1"} produced in example 4 is used in a new process as described in example 1 excepted the raw materials methyl acrylate, dimethylaminoethanol are adjusted referring to the residual monomer in the organic solution OS_{1"} and that half of OS_{1"} is replaced per its equivalent amount of fresh DBTO based on Sn content. The operation is repeated 10 times. The results are summarized in table 5.

| **Example** | **10** | **10.1** | **10.2** | **10.3** | **10.4** | **10.5** | **10.6** | **10.7** | **10.8** | **10.9** |
|---|---|---|---|---|---|---|---|---|---|---|
| **Number of synthesis** | 1 (example 1) | 2 | 3 | 4 | 5 | 6 | 7 | 8 | 9 | 10 |
| **ADAME conversion based on DMOH %** | 98.2 | 94.5 | 95.2 | 95.3 | 94.6 | 94.7 | 94.0 | 94.0 | 94.1 | 93.9 |
| **Hv in reactor prior ADAME distillation %** | 0.35 | 0.80 | 1.5 | 2.3 | 2.9 | 3.2 | 3.5 | 3.8 | 4.0 | 4.1 |
| **ADAME purity after distillation %** | 99.5 | 99.3 | 99.6 | 99.0 | 99.3 | 99.3 | 99.3 | 99.3 | 99.3 | 99.3 |
| **Hv in ADAME fraction ppm** Table 5 | 505 | 525 | 580 | 670 | 750 | 820 | 850 | 910 | 950 | 995 |

### Example 11 - ADAME polymer synthesis and application tests

**Table 6**

| **Polymer obtained from monomer produced in ex n°** | **8.1** | **8.2** | **8.3** | **8.4** | **8.5** | **8.6** | **8.7** | **8.8** | **8.9** |
|---|---|---|---|---|---|---|---|---|---|
| **UL viscosity (cps)** | 5.35 | 5.15 | 5.1 | 5.00 | 4.95 | 4.82 | 4.70 | Polymerization inhibited | |
| **Insoluble number** | 0 | 0 | 20 | 30 | 30 | Clogged filter | clogged filter | | |

ADAME monomer of examples 8, 9 and 10 have been quaternized and polymer prepared according to example 7. The UL viscosity and the insoluble number have been compared to see the effect of the different recycling method according to the invention. The results are summarized in table 6, 7 and 8.

**Table 7**

| **Polymer obtained from monomer produced in ex n°** | **9.1** | **9.2** | **9.3** | **9.4** | **9.5** | **9.6** | **9.7** | **9.8** | **9.9** |
|---|---|---|---|---|---|---|---|---|---|
| **UL viscosity (cps)** | 5.3 | 5.25 | 5.30 | 5.25 | 5.35 | 5.10 | 5.15 | 4.89 | 4.76 |
| **Insoluble number** | 0 | 3 | 2 | 3 | 5 | 5 | 5 | 10 | 20 |

**Table 8**

| **Polymer obtained from monomer produced in ex n°** | **10.1** | **10.2** | **10.3** | **10.4** | **10.5** | **10.6** | **10.7** | **10.8** | **10.9** |
|---|---|---|---|---|---|---|---|---|---|
| **UL viscosity (cps)** | 5.3 | 5.15 | 5.30 | 5.10 | 5.25 | 5.10 | 5.15 | 5.25 | 5.30 |
| **Insoluble number** | 0 | 3 | 2 | 5 | 5 | 3 | 5 | 5 | 6 |

## Claims

1. Recycling method of an organotin catalyst residue, said recycling method comprising the following successive steps:
1) Preparation of a mixture comprising (i) a polar solvent selected from water, at least one C₁-C₁₂ alcohol, and a mixture of water and at least one C₁-C₁₂ alcohol (ii) an organotin catalyst residue solution S₁ resulting from the synthesis of an amino alkyl (meth)acrylate compound and (iii) at least one organic extraction solvent ES₁, liquid-liquid extraction LLE₁ of this mixture using the organic extraction solvent ES₁ to obtain an organic solution OS₁ and an aqueous solution AS₁;
2) Distillation D₁, at a temperature T₁ and at a pressure P₁, of the organic solution OS₁ to obtain an organic solution OS_{1'} and an organic distillate OD₁;
3) Distillation D₂, at a temperature T₂ and at a pressure P₂, of the organic solution OS_{1'} to obtain an organic solution OS_{1"} and and an organic distillate OD₂, wherein the organic solution OS_{1"} contains 2 to 40 wt% of organotin catalyst residue;
the temperature T₂ being greater than the temperature T₁ and/or the pressure P₂ being lower than the pressure P₁;
wherein in step 1), the (ii) organotin catalyst residue solution S₁ and the (iii) at least organic extraction solvent ES₁ are already mixed when the (i) polar solvent is added or the (i) polar solvent, the (ii) organotin catalyst residue solution S₁ and the (iii) at least organic extraction solvent ES₁ are added simultaneously;
and the organic extraction solvent ES₁ and the polar solvent have a weight ratio ES₁:polar solvent comprised between 20:1 and 2:1;
wherein distillation D₁ is carried out at a temperature T₁ comprised between 5 and 180°C, and;
wherein distillation D₁ is carried out at an absolute pressure P₁ comprised between 0.01 and 1 bar.

2. Recycling method according to claim 1, wherein the organotin catalyst residue solution S₁ results from the synthesis of 2-dimethylaminoethyl (meth)acrylate.

3. Recycling method according to claim 2, wherein the organotin catalyst residue solution S₁ results from the synthesis of 2-dimethylaminoethyl (meth)acrylate in the presence of dialkyl tin oxide.

4. Recycling method according to claim 3, wherein dialkyl tin oxide is dibutyl tin oxide.

5. Recycling method according to claim 1, wherein the organic extraction solvent ES₁ is selected from linear and saturated hydrocarbons containing from 3 to 16 carbons, linear and unsaturated hydrocarbons containing from 3 to 16 carbons, branched and saturated hydrocarbons containing from 3 to 16 carbons, branched and unsaturated hydrocarbons containing from 3 to 16 carbons, cyclic and saturated hydrocarbons containing from 3 to 16 carbons, cyclic and unsaturated hydrocarbons containing from 3 to 16 carbons and mixtures thereof.

6. Recycling method according to claim 1, wherein the organic extraction solvent ES₁ contains at least 50 weight % of n-hexane.

7. Recycling method according to claim 1, wherein the polar solvent contains at least 80 weight % of water.

8. Recycling method according to claim 1, wherein the liquid-liquid extraction LLE₁ comprises at least one mixing step and one separation step.

9. Recycling method according to claim 8, wherein the mixing step lasts between 5 seconds and 60 minutes, and involves a mechanical stirring having a rotation speed of between 10 and 10 000 rpm.

10. Recycling method according to claim 1, wherein distillation D₂ is carried out at a temperature T₂ comprised between 20 and 220°C.

11. Recycling method according to claim 1, wherein distillation D₂ is carried out at an absolute pressure P₂ comprised between 0.001 and 1 bar.

12. Recycling method according to claim 1, wherein the recycling method includes one or more of the following additional liquid-liquid extraction LLEₙ₊₁, wherein n is an integer greater than or equal to 1:
- Preparation of a mixture comprising (i) an aqueous solution ASₙ (ii) an organotin catalyst residue solution S₁ resulting from the synthesis of an amino alkyl (meth)acrylate compound and (iii) at least one least an organic extraction solvent ESₙ,
liquid-liquid extraction LLEₙ₊₁ of this mixture to obtain an organic solution OSₙ₊₁ and aqueous solution ASₙ₊₁.

13. Recycling method according to claim 12, wherein at least part of the organic solution OSₙ₊₁ is distilled according to steps 2) and 3) of claim 1, to respectfully afford an organic solution OS'ₙ₊₁ and then an organic solution OS''ₙ₊₁.

14. Recycling method according to claim 12, wherein the aqueous solution ASₙ is used partially or totally, with or without pre-treatment step for a liquid-liquid extraction LLEₙ₊₁ and the organic solution OSₙ₊₁ is recycled partially or totally, with or without pre-treatment step in a liquid-liquid extraction LLEₙ.

15. Recycling method according to claim 1, wherein distillation D₁ and distillation D₂ are carried out in the same distillation device.

16. Recycling method according to claim 1, wherein the method is carried out as a continuous process.

## Patentansprüche

1. Recyclingverfahren für einen Organozinn-Katalysatorrückstand, wobei das genannte Recyclingverfahren die folgenden aufeinanderfolgenden Schritte umfasst:
1) Zubereitung eines Gemischs, umfassend (i) ein polares Lösungsmittel, ausgewählt aus Wasser, zumindest einem C₁-C₁₂-Alkohol und einer Mischung aus Wasser und zumindest einem C₁-C₁₂-Alkohol, (ii) eine Organozinn-Katalystorrückstandslösung S₁, die aus der Synthese einer Aminoalkyl(meth)acrylatverbindung resultiert, und (iii) zumindest ein organisches Extraktionslösungsmittel ES₁, Flüssig-Flüssig-Extraktion LLE₁ dieses Gemischs unter Verwendung des organischen Extraktionslösungsmittels ES₁, um eine organische Lösung OS₁ und eine wässrige Lösung AS₁ zu erhalten;
2) Destillation D₁ bei einer Temperatur T₁ und einem Druck P₁ der organischen Lösung OS₁, um eine organische Lösung OS_{1'} und ein organisches Destillat OD₁ zu erhalten;
3) Destillation D₂ bei einer Temperatur T₂ und einem Druck P, der organischen Lösung OS_{1'}, um eine organische Lösung OS_{1"} und ein organisches Destillat OD₂ zu erhalten, worin die organische Lösung OS_{1"} 2 bis 40 Gew.-% Organozinn-Katalysatorrückstand enthält;
wobei die Temperatur T₂ höher ist als die Temperatur T₁ und/oder der Druck P₂ niedriger ist als der Druck P₁;
worin in Schritt 1) die (ii) Organozinn-Katalysatorrückstandslösung S₁ und das (iii) zumindest organische Extraktionslösungsmittel ES₁ bereits gemischt sind, wenn das (i) polare Lösungsmittel zugefügt wird, oder das (i) polare Lösungsmittel, die (ii) Organozinn-Katalysatorrückstandslösung S₁ und das (iii) zumindest organische Extraktionslösungsmittel ES₁ gleichzeitig zugefügt werden;
und das organische Extraktionslösungsmittel ES₁ und das polare Lösungsmittel ein Gewichtsverhältnis ES₁:polarem Lösungsmittel aufweisen, das zwischen 20:1 und 2:1 liegt;
worin die Destillation D₁ bei einer Temperatur T₁ durchgeführt wird, die zwischen 5 und 180 °C liegt, und;
worin die Destillation D₁ bei einem absoluten Druck P₁ durchgeführt wird, der zwischen 0,01 und 1 bar liegt.

2. Recyclingverfahren nach Anspruch 1, worin die Organozinn-Katalysatorrückstandslösung S₁ aus der Synthese von 2-Dimethylaminoethyl(meth)acrylat resultiert.

3. Recyclingverfahren nach Anspruch 2, worin die Organozinn-Katalysatorrückstandslösung S₁ aus der Synthese von 2-Dimethylaminoethyl(meth)acrylat bei Vorhandensein von Dialkylzinnoxid resultiert.

4. Recyclingverfahren nach Anspruch 3, worin Dialkylzinnoxid Dibutylzinnoxid ist.

5. Recyclingverfahren nach Anspruch 1, worin das organische Extraktionslösungsmittel ES₁ aus linearen und gesättigten Kohlenwasserstoffen mit 3 bis 16 Kohlenstoffen, linearen und ungesättigten Kohlenwasserstoffen mit 3 bis 16 Kohlenstoffen, verzweigten und gesättigten Kohlenwasserstoffen mit 3 bis 16 Kohlenstoffen, verzweigten und ungesättigten Kohlenwasserstoffen mit 3 bis 16 Kohlenstoffen, zyklischen und gesättigten Kohlenwasserstoffen mit 3 bis 16 Kohlenstoffen, zyklischen und ungesättigten Kohlenwasserstoffen mit 3 bis 16 Kohlenstoffen und Gemischen davon ausgewählt wird.

6. Recyclingverfahren nach Anspruch 1, worin das organische Extraktionslösungsmittel ES₁ zumindest 50 Gew.-% n-Hexan enthält.

7. Recyclingverfahren nach Anspruch 1, worin das polare Lösungsmittel zumindest 80 Gew.-% Wasser enthält.

8. Recyclingverfahren nach Anspruch 1, worin die Flüssig-Flüssig-Extraktion LLE₁ zumindest einen Mischungsschritt und einen Trennungsschritt umfasst.

9. Recyclingverfahren nach Anspruch 8, worin der Mischungsschritt zwischen 5 Sekunden und 60 Minuten dauert und ein mechanisches Rühren mit einer Drehgeschwindigkeit von zwischen 10 und 10.000 U/min umfasst.

10. Recyclingverfahren nach Anspruch 1, worin die Destillation D₂ bei einer Temperatur T₂ zwischen 20 und 220 °C durchgeführt wird.

11. Recyclingverfahren nach Anspruch 1, worin die Destillation D₂ bei einem absoluten Druck P₂ zwischen 0,001 und 1 bar durchgeführt wird.

12. Recyclingverfahren nach Anspruch 1, worin das Recyclingverfahren einen oder mehrere der folgenden zusätzlichen Flüssig-Flüssig-Extraktionen LLEₙ₊₁ beinhaltet, worin n eine ganze Zahl größer oder gleich 1 ist:
- Zubereitung eines Gemischs, umfassend (i) eine wässrige Lösung ASₙ (ii) eine Organozinn-Katalysatorrückstandslösung S₁, die aus der Synthese einer Aminoalkyl(meth)acrylat-Verbindung resultiert, und (iii) zumindest ein organisches Extraktionslösungsmittel ESₙ, Flüssig-Flüssig-Extraktion LLEₙ₊₁ dieses Gemischs, um eine organische Lösung OSₙ₊₁ und wässrige Lösung ASₙ₊₁ zu erhalten.

13. Recyclingverfahren nach Anspruch 12, worin zumindest ein Teil der organischen Lösung OSₙ₊₁ entsprechend den Schritten 2) und 3) in Anspruch 1 destilliert wird, um jeweils eine organische Lösung OS'ₙ₊₁ und dann eine organische Lösung OS_{"2+1} zu erhalten.

14. Recyclingverfahren nach Anspruch 12, worin die wässrige Lösung ASₙ teilweise oder vollständig mit oder ohne Vorbehandlungsschritt für eine Flüssig-Flüssig-Extraktion LLEₙ₊₁ verwendet wird, und die organische Lösung OSₙ₊₁ teilweise oder vollständig mit oder ohne Vorbehandlungsschritt in einer Flüssig-Flüssig-Extraktion LLEₙ recycelt wird.

15. Recyclingverfahren nach Anspruch 1, worin Destillation D₁ und Destillation D₂ in derselben Destillationsvorrichtung durchgeführt werden.

16. Recyclingverfahren nach Anspruch 1, worin das Verfahren als kontinuierlicher Prozess durchgeführt wird.

## Revendications

1. Procédé de recyclage d'un résidu de catalyseur à base d'organoétain, ledit procédé de recyclage comprenant les étapes successives suivantes :
1) préparation d'un mélange comprenant (i) un solvant polaire choisi parmi l'eau, au moins un alcool en C₁-C₁₂ et un mélange d'eau et d'au moins un alcool en C₁-C₁₂ (ii) une solution de résidu de catalyseur à base d'organoétain S₁ résultant de la synthèse d'un composé de (méth)acrylate d'aminoalkyle et (iii) au moins un solvant d'extraction organique ES₁, une extraction liquide-liquide LLE₁ de ce mélange en utilisant le solvant d'extraction organique ES₁ pour obtenir une solution organique OS₁ et une solution aqueuse AS₁ ;
2) distillation D₁, à une température T₁ et à une pression P₁, de la solution organique OS₁ pour obtenir une solution organique OS₁'et un distillat organique OD₁ ;
3) distillation D₂, à une température T₂ et à une pression P₂, de la solution organique OS_{1'}pour obtenir une solution organique OS_{1"} et un distillat organique OD₂, dans lequel la solution organique OS_{1"} contient de 2 à 40 % en poids de résidu de catalyseur à base d'organoétain ;
la température T₂ étant supérieure à la température T₁ et/ou la pression P₂ étant inférieure à la pression P₁ ;
dans lequel à l'étape 1), la (ii) solution de résidu de catalyseur à base d'organoétain S₁ et le (iii) au moins un solvant d'extraction organique ES₁ sont déjà mélangés lorsque le (i) solvant polaire est ajouté ou le (i) solvant polaire, la (ii) solution de résidu de catalyseur à base d'organoétain S₁ et le (iii) au moins un solvant d'extraction organique ES₁ sont ajoutés simultanément ;
et le solvant d'extraction organique ES₁ et le solvant polaire présentent un rapport pondéral ES₁:solvant polaire compris entre 20:1 et 2:1 ;
dans lequel la distillation D₁ est effectuée à une température T₁ comprise entre 5 et 180°C, et ;
dans lequel la distillation D₁ est effectuée à une pression absolue P₁ comprise entre 0,01 et 1 bar.

2. Procédé de recyclage selon la revendication 1, dans lequel la solution de résidu de catalyseur à base d'organoétain S₁ résulte de la synthèse de (méth)acrylate de 2-diméthylaminoéthyle.

3. Procédé de recyclage selon la revendication 2, dans lequel la solution de résidu de catalyseur à base d'organoétain S₁ résulte de la synthèse de (méth)acrylate de 2-diméthylaminoéthyle en présence d'oxyde de dialkyle étain.

4. Procédé de recyclage selon la revendication 3, dans lequel l'oxyde de dialkyle étain est l'oxyde de dibutyle étain.

5. Procédé de recyclage selon la revendication 1, dans lequel le solvant d'extraction organique ES₁ est choisi parmi des hydrocarbures linéaires et saturés contenant de 3 à 16 atomes de carbone, des hydrocarbures linéaires et insaturés contenant de 3 à 16 atomes de carbone, des hydrocarbures ramifiés et saturés contenant de 3 à 16 atomes de carbone, des hydrocarbures ramifiés et insaturés contenant de 3 à 16 atomes de carbone, des hydrocarbures cycliques et saturés contenant de 3 à 16 atomes de carbone, des hydrocarbures cycliques et insaturés contenant de 3 à 16 atomes de carbone, et des mélanges de ceux-ci.

6. Procédé de recyclage selon la revendication 1, dans lequel le solvant d'extraction organique ES₁ contient au moins 50 % en poids de n-hexane.

7. Procédé de recyclage selon la revendication 1, dans lequel le solvant polaire contient au moins 80 % en poids d'eau.

8. Procédé de recyclage selon la revendication 1, dans lequel l'extraction liquide-liquide LLE₁ comprend au moins une étape de mélange et une étape de séparation.

9. Procédé de recyclage selon la revendication 8, dans lequel l'étape de mélange dure entre 5 secondes et 60 minutes et implique une agitation mécanique présentant une vitesse de rotation comprise entre 10 et 10 000 tr/min.

10. Procédé de recyclage selon la revendication 1, dans lequel la distillation D₂ est effectuée à une température T₂ comprise entre 20 et 220°C.

11. Procédé de recyclage selon la revendication 1, dans lequel la distillation D₂ est effectuée à une pression absolue P₂ comprise entre 0,001 et 1 bar.

12. Procédé de recyclage selon la revendication 1, dans lequel le procédé de recyclage inclut une ou plusieurs de l'extraction liquide-liquide supplémentaire suivante LLEₙ₊₁, dans lequel n est un entier supérieur ou égal à 1 :
- préparation d'un mélange comprenant (i) une solution aqueuse Asₙ, (ii) une solution de résidu de catalyseur à base d'organoétain S₁ résultant de la synthèse d'un composé de(méth)acrylate d'aminoalkyle et (iii) au moins un solvant d'extraction organique Esₙ, une extraction liquide-liquide LLEₙ₊₁ de ce mélange pour obtenir une solution organique OSₙ₊₁ et une solution aqueuse ASₙ₊₁.

13. Procédé de recyclage selon la revendication 12, dans lequel au moins une partie de la solution organique OSₙ₊₁ est distillée selon les étapes 2) et 3) de la revendication 1, pour obtenir respectueusement une solution organique OS_{'n+1}, puis une solution organique OS_{"n+1}.

14. Procédé de recyclage selon la revendication 12, dans lequel la solution aqueuse ASₙ est utilisée partiellement ou totalement, avec ou sans étape de prétraitement pour une extraction liquide-liquide LLEₙ₊₁ et la solution organique OSₙ₊₁ est recyclée partiellement ou totalement, avec ou sans étape de prétraitement, dans une extraction liquide-liquide LLEₙ.

15. Procédé de recyclage selon la revendication 1, dans lequel la distillation D₁ et la distillation D₂ sont effectuées dans le même dispositif de distillation.

16. Procédé de recyclage selon la revendication 1, dans lequel le procédé est mis en œuvre sous la forme d'un processus continu.
